# EUROPÄISCHE PATENTSCHRIFT

(11) **EP 1 354 610 B1**
(45) Veröffentlichungstag und Bekanntmachung des Hinweises auf die Patenterteilung: **30.03.2005**
(21) Anmeldenummer: 03090109.4
(22) Anmeldetag: 15.04.2003
(51) Int. Cl.: A61N 1/05, H01B 1/12

(54) **Elektrodenleitung aus einem intrinsisch leitfähigen Polymer**
Electrode lead made of an intrinsically conductive polymer
Fil d'électrode constitué d'un polymère électriquement conducteur

(30) Priorität: 16.04.2002 DE 10217828
(43) Veröffentlichungstag der Anmeldung: 22.10.2003
(73) Patentinhaber: BIOTRONIK Mess- und Therapiegeräte GmbH & Co Ingenieurbüro Berlin, 12359 Berlin (DE)
(72) Erfinder: Kranz, Curt, 14163 Berlin (DE); Kolberg, Gernot, 12043 Berlin (DE)
(74) Vertreter: Eisenführ, Speiser & Partner

(56) Entgegenhaltungen:
- DE-A- 2 822 829
- US-A- 5 476 496
- US-A- 5 554 176
- US-A- 5 681 514
- PATENT ABSTRACTS OF JAPAN vol. 010, no. 310 (C-379), 22. Oktober 1986 (1986-10-22) & JP 61 118454 A (HITACHI LTD), 5. Juni 1986 (1986-06-05)

## Beschreibung

Die vorliegende Erfindung betrifft eine Elektrodenleitung für eine implantierbare, intravaskuläre Elektrostimulationsvorrichtung mit den im Oberbe-griff des Anspruchs 1 genannten Merkmalen.

Elektrodenleitungen, die beispielsweise in Blutgefäße eingeführt werden oder durch Blutgefäße hindurch in eine Kammer eines Herzens führen, sind grundsätzlich bekannt. Solche Elektrodenleitungen tragen im Allgemeinen Elektroden, die dazu dienen umliegendes Körpergewebe elektrisch anzuregen oder elektrische Signale aufzunehmen. Bekannt sind beispielsweise Stimulationselektroden für Herzschrittmacher.

Gängige Herzschrittmacher umfassen dazu zumindest einen Impulsgeber mit einer Spannungsquelle, der über die Elektrodenleitung mit den mit dem Herzgewebe in Verbindung stehenden Elektroden leitend verbunden ist.

Moderne Impuls- und Detektionsverfahren basieren in der Regel auf Mehrfachanordnungen von Elektroden, die simultan oder nach einem vorgebbaren Muster angesteuert werden. Während der Stimulation beziehungsweise Detektion wechseln häufig Polarität und Höhe der angelegten Spannung drastisch. Ein Teil der Elektrodenleitungen kann unter Umständen auch zur Ansteuerung von Sensoren genutzt werden, die physiologische Messwerte wie die Sauerstoffkonzentration, die Bluttemperatur und den Blutdruck erfassen. Alle diese Funktionselemente (Elektroden, Sensoren) werden üblicherweise über metallische Leiter mit dem Herzschrittmacher verbunden. Elektrodenleitungen auf Basis metallischer Leiter sind jedoch bruchempfindlich, was insbesondere im Zusammenhang mit der stetigen Kontraktionsbewegung des Herzens problematisch werden kann. Zudem wächst mit einer zunehmenden Anzahl von Funktionselementen ein Durchmesser des Kabelstranges der für die Spannungsversorgung beziehungsweise Ansteuerung der Funktionselemente benötigt wird. Damit einhergehend erhöht sich die Steifheit der Elektrodenleitung, was sich wiederum ungünstig bei der Implantation der Elektrostimulationsvorrichtung und ihre dauerhafte Verträglichkeit auswirkt.

Aus der US 5,476,496 von Standberg et al. ist ein implantierbares Elektrodensystem bekannt, das eine indifferente Elektrode aus einem elektrisch leitfähigen Polymer beinhaltet. Die Elektrode aus dem elektrisch leitfähigem Polymer soll eine möglichst große Kontaktoberfläche zum Gewebe des Herzens bereitstellen.

Maddison et al. (US 5,554,176) offenbart Elektrodenteitungen, die sowohl für die Spannungsversorgung von Elektroden, als auch für die Ansteuerung von Sensoren verwendet werden können. Als elektrische Leiter dienen unter anderen koaxial verlaufende, von einem Isolator ummantelte Bereiche aus einem leitfähigen Polymer. Diese leitfähigen Bereiche der Elektrodenleitung können konzentrisch um die Längsachse der Elektrodenleitung verlaufen oder sie bestehen aus einzelnen Strängen die miteinander verwoben oder isoliert zu dem jeweiligen Funktionselement verlaufen. Gemeinsam ist diesen Anordnungen, dass sich elektrisch leitfähige und isolierende Bereiche abwechseln. Dies erfordert relativ komplexe und damit kostenintensive Herstellungsverfahren.

Aufgabe der vorliegenden Erfindung ist es, eine Elektrodenleitung zur Verfügung zu stellen, die in ihrer Herstellung besonders einfach ist und die es erlaubt eine Vielzahl von Funktionselementen simultan oder in vorgebbarer Weise anzusteuern.

Die Aufgabe wird durch die Elektrodenleitung für eine implantierbare, intravaskuläre Elektrostimulationsvorrichtung mit den im Anspruch 1 genannten Merkmalen gelöst. Sie zeichnet sich dadurch aus, die Elektrodenleitung aus einem intrinsisch leitfähigen Polymer besteht, bei dem die einzelnen Polymerketten des Polymers so ausgerichtet sind, dass eine hohe elektrische Leitfähigkeit in axialer nicht jedoch in radialer Richtung der Elektrodenleitung besteht (elektrische Anisotropie). Die Elektrodenleitung hat damit im Querschnitt einen weitestgehend homogenen Aufbau, was die Herstellung wesentlich vereinfacht.

Als intrinsisch leitfähige Polymere werden vorzugsweise Polymere aus Polyacetylenen (PAC), Polyparaphenylen (PPP), Polyphenylensulfid (PPS), Polyparaphenylvinylen (PPV), Polyphenylenbutadien (PPB), Polyparapyridin (PPYR), Polypyrrol (PPY), Polyfuran (PFU), Polythiophen (PT), Polyphenylamin (PANI), Polyethylendioxythiophen (PEDT), Polyethylendioxythophen-Polystyrolsulfonat (PEDT/PSS), Polyacen eingesetzt. Die Materialien zeichnen sich dadurch aus, dass sie unter geeigneten Polymerisations- und Verarbeitungsbedingungen im zu erstellenden Werkstück axial ausgerichtete Polymerketten ausbilden. Ein spezifischer Durchgangswiderstand einer Elektrodenleitung auf Basis eines solchen intrinsisch leitfähigen Polymers ist vorzugsweise in radialer Richtung größer 5*10⁻¹ Ωm, insbesondere größer 1 Ωm. Parallel zur Längsachse der Elektrodenleitung, also in axialer Richtung, beträgt der spezifische Durchgangswiderstand der Elektrodenleitung vorzugsweise 10⁻² bis 10⁻⁶ Ωm, insbesondere 10⁻⁴ bis 10⁻⁶ Ωm. Die anisotrope elektrische Leitfähigkeit der Materialien ist an sich bekannt, sodass auf eine ausführliche Beschreibung dieser Eigenschaft und der Herstellung der Polymere verzichtet werden kann.

Weiterhin ist bevorzugt, dass die Elektrodenleitung in ihrem Querschnitt in einzelne Sektoren gegliedert ist, die voneinander unabhängig in axialer Richtung elektrisch leitfähig sind. Die Sektoren können dabei vorzugsweise als Umlaufsegmente oder radiale Kreisausschnitte ausgebildet sein. Auf diese Weise kann erreicht werden, dass beim Anlegen einer elektrischen Spannung ein elektrischer Strom nur zwischen den korrespondierenden Sektoren am proximalen und distalen Ende der Elektrodenleitung fließt.

Die Elektrodenleitung ist ferner bevorzugt von einer biokompatiblen, Isolierhülle ummantelt. Als zu kontaktierende Funktionselemente im distalen Bereich der Elektrodenleitung kommen Elektroden sowie Sensoren zur Bestimmung physiologischer Parameter in Frage.

Ferner hat es sich als vorteilhaft erwiesen, wenn am proximalen Ende der Elektrodenleitung Kontaktelemente der Elektrostimulationsvorrichtung einem oder mehreren Sektoren zugeordnet sind. Ein einem bestimmten Sektor beziehungsweise mehreren Sektoren zugeordnetes Kontaktelement gewährt dann insbesondere einen elektrischen Kontakt zu den am distalen Ende der Elektrodenleitung angeordneten Funktionselementen desgleichen beziehungsweise dergleichen Sektoren. Es können demnach komplexe Leitungsgeometrien in einer einzigen Elektrodenleitung realisiert werden.

Weitere bevorzugte Ausgestaltungen der Erfindung ergeben sich aus den übrigen, in den Unteransprüchen genannten Merkmalen.

Die Erfindung wird nachfolgend anhand von Zeichnungen näher erläutert. Es zeigen:
- Figur 1:: eine Prinzipdarstellung einer Elektrostimulationsvorrichtung mit Elektrodenleitung;
- Figur 2:: die Elektrodenleitung mit Querschnitten an ihrem proximalen und distalen Ende und
- Figur 3:: eine schematische Veranschaulichung des aniostropen elektrischen Verhaltens der Elektrodenleitung.

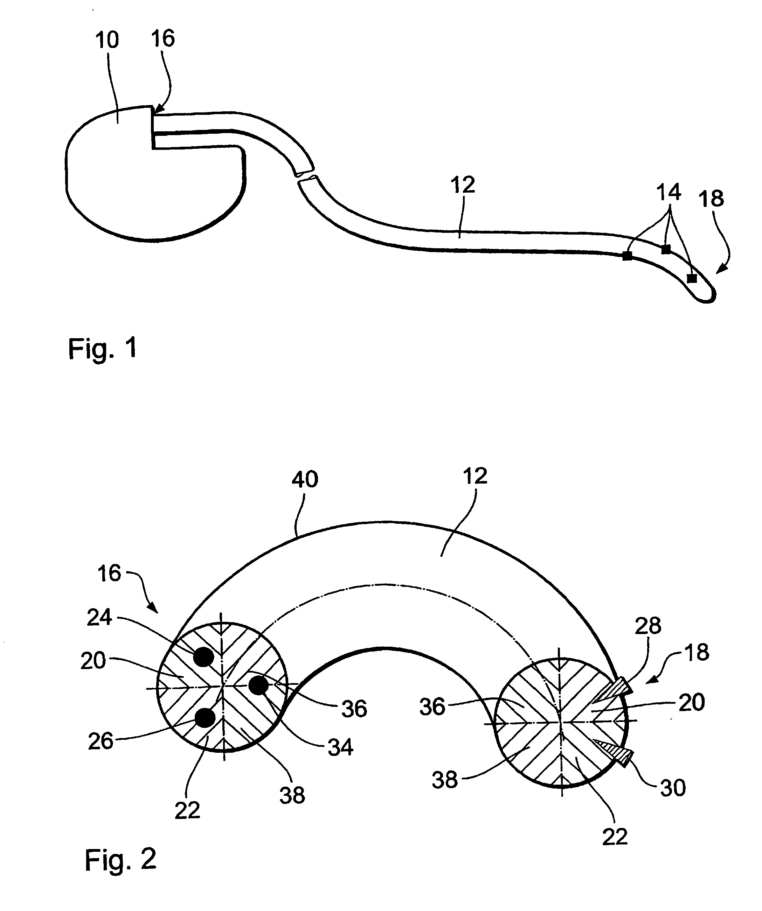

In der Figur 1 ist der prinzipielle Aufbau einer Elektrostimulationsvorrichtung 10 mit einer Elektrodenleitung 12 vereinfacht dargestellt. Die Elektrostimulationsvorrichtung 10, beispielsweise in Form eines Herzschrittmachers, beinhaltet unter anderem Komponenten, die als Spannungsquelle, Steuer- und Auswerteeinheit und Telemetrieeinheit dienen. Der prinzipielle Aufbau derartiger Elektrostimulationsvorrichtungen 10 ist seit langem bekannt. Ebenso bekannt ist es, die Elektrostimulationsvorrichtung 10 in intravaskuläres Gewebe zu implantieren.

Die Elektrodenleitung 12 ermöglicht einen elektrischen Kontakt zwischen der Elektrostimulationsvorrichtung 10 und einem am distalen Ende 18 der Elektrodenleitung 12 angeordneten Funktionselement 14. Mit ihrem proximalen Ende 16 mündet die Elektrodenleitung 12 in geeigneter Weise in die Elektrostimulationsvorrichtung 10. Die einzelnen Funktionselemente 14 können dabei als Elektroden zum Stimulieren oder Abfühlen von Körpergewebe ausgebildet sein und/oder sie umfassen Sensoren zur Bestimmung physiologischer Parameter. Letztere können beispielsweise Blutdruck, Bluttemperatur oder Sauerstoffpartialdruck erfassen. Gemeinsam ist den Funktionselementen 14, dass sie zur Durchführung der für sie vorgesehenen Aktivitäten elektrisch leitend mit der Elektrostimulationsvorrichtung 10 verbunden sein müssen. Die Elektrostimulationsvorrichtung 10 steuert diese Aktivitäten der Funktionselemente 14 mittels ihrer Auswerte- und Steuereinheit.

Die Ansteuerung der einzelnen Funktionselemente 14 durch die Elektrostimulationsvorrichtung 10 kann simultan oder nach einem vorgebbaren Muster erfolgen. Zu den Einzelheiten hierzu wird auf den umfangreichen Stand der Technik verwiesen. Festzuhalten bleibt an dieser Stelle nur, dass die Funktionselemente 14 nach Möglichkeit separat oder zumindest in separaten Gruppen ansteuerbar sein sollen. Dazu muss die Elektrodenleitung 12 entsprechend differenzierte, elektrisch leitfähige Bereiche aufweisen.

Die Figur 2 zeigt die Elektrodenleitung 12 mit einem Querschnitt jeweils an ihrem proximalen und distalen Ende 16, 18. Am distalen Ende 18 sind als Funktionselemente 14 eine erste Elektrode 28 und eine zweite Elektrode 30 vorgesehen. In dem Bereich, in dem das proximale Ende 16 der Elektrodenleitung 12 mit der Etektrostimutationsvomchtung 10 in Kontakt tritt, weist diese ein erstes und zweites Kontaktelement 24, 26 auf. Die Kontaktelemente stehen in nicht dargestellter, aber an sich bekannter Weise mit der Spannungsquelle beziehungsweise Auswerte- und Steuereinheit der Elektrostimulationsvorrichtung 10 in Verbindung. Das erste Kontaktelement 24 und die erste Elektrode 28 liegen in einem gemeinsamen ersten Sektor 20, dessen radiale Erstreckung durch das Kreissegment angedeutet ist. In einem zweiten Sektor 22 sind entsprechend das zweite Kontaktelement 26 und die zweite Elektrode 30 angeordnet.

Neben der hier dargestellten Aufteilung des Querschnitts der Elektrodenleitung 12 in insgesamt vier radiale Kreisausschnitte sind auch Ausgestaltungen denkbar, bei denen die weiter unten näher erläuterten Sektoren 20, 22 Umlaufsegmente um eine Längsachse der Elektrodenleitung 12 bilden. Die Sektoren 20, 22 basieren jeweils auf einem intrinsisch leitfähigem Polymer, welches aus vorherrschend in axialer Richtung orientierten Polymerketten besteht.

Derartige Polymere besitzen in der Regel ausgedehnte C=C-Doppelbindungssysteme, die chemisch leicht angreifbar sind. Dies passiert beim Dotieren. Eine Zugabe von Elektronendonatoren (Natrium, Kalium, Cäsium) oder Elektronenakzeptoren (I₂, SbCl₅, FeCl₃ oder ähnliches) führt zu einer erhöhten Elektronenbeweglichkeit und zu Leitfähigkeiten von bis zu 10⁵ S/cm. Als intrinsisch leitfähige Polymere haben sich als bevorzugt Polymere aus Polyacetylenen (PAC), Polyparaphenylen (PPP), Polyphenylensulfid (PPS), Polyparaphenylvinylen (PPV), Polyphenylenbutadien (PPB), Polyparapyridin (PPYR), Polypyrrol (PPY), Polyfuran (PFU), Polythiophen (PT), Polyphenylamin (PANI), Polyethylendioxythiophen (PEDT), Polyethylendioxythophen-Polystyrolsulfonat (PEDT/PSS), Polyacen erwiesen. Durch gezielte Wahl der Reaktionsbedingungen lässt sich die Polymerisation der jeweils eingesetzten Monomere derart steuern, dass der resultierende Werkstoff bei geeigneter Verarbeitung ein elektrisch anisotropes Verhalten zeigt. Dabei kann auf bekannte Kunststoffverarbeitungsverfahren zurückgegriffen werden. Mit Vorgabe der jeweils den Einzelfall anzupassenden Polymerisations- und Verarbeitungsbedingungen kann sichergestellt werden, dass die einzelnen Polymerketten des intrinsisch leitfähigen Polymers in Längsrichtung der Elektrodenleitung 12 koaxial ausgerichtet sind und keine nennenswerte Leitfähigkeit in radialer Richtung entsteht.

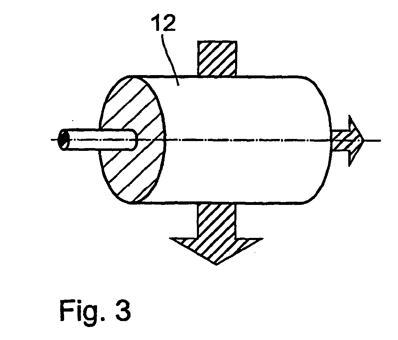

Ein spezifischer Durchgangswiderstand der Elektrodenleitung (12) auf Basis eines solchen intrinsisch leitfähigen Polymers ist in radialer Richtung größer 5*10⁻¹ Ωm und beträgt in axialer, also paralleler Erstreckung zur Längsachse der Elektrodenleitung (12), 10⁻² bis 10⁻⁸ Ωm. Je größer die Unterschiede zwischen den axialen und radialen spezifischen Durchgangswiderständen sind, desto besser lassen sich Fehlschaltungen vermeiden. Ein besonders günstiges anisotropes elektrisches Verhalten liegt vor, wenn der spezifische Durchgangswiderstand in radialer Richtung größer 1 Ωm ist und in axialer Richtung zwischen 10⁻⁴ bis 10⁻⁶ Ωm liegt. Die Figur 3 soll das Phänomen der elektrischen Anisotropie noch einmal verdeutlichen. Der geringe axiale spezifische Durchgangswiderstand ist durch den parallel zu Längsachse der Elektrodenleitung 12 verlaufenden schmalen Pfeil angedeutet. Senkrecht hierzu steht der fette, einen großen spezifischen Durchgangswiderstand repräsentierende Pfeil.

Mit Hilfe eines geeigneten Kunststoffverarbeitungsverfahrens lässt sich die Elektrodenleitung 12 in ihrem Querschnitt in die bereits erwähnten Sektoren 20, 22 gliedern. Durch die separate Darstellung der Sektoren 20, 22 kann wirkungsvoll verhindert werden, dass Polymerketten sich über mehrere Sektoren 20, 22 erstrecken. Wird nun beispielsweise an das erste Kontaktelement 24 eine Spannung angelegt, so kann sich ein elektrischer Kontakt nur zu der ersten Elektrode 28 im Sektor 20 aufbauen. Soll die zweite Elektrode 30 angesteuert werden, so muss demnach eine Kontaktierung über das im gleichen Sektor 22 liegende zweite Kontaktelement 26 erfolgen.

Denkbar ist auch, dass sich ein Kontaktelement am proximalen Ende 16 der Elektrodenleitung 12 über mehrere Sektoren erstreckt und so die gleichzeitige Ansteuerung mehrerer Elektroden und/oder Sensoren ermöglicht wird. Ein Beispiel hierfür ist ein Kontaktelement 34, welches sich sowohl über den Sektor 36 als auch den Sektor 38 erstreckt. Im Bereicht der Sektoren 36, 38 liegende Funktionselemente - hier nicht dargestellt - können auf diese Weise simultan angesteuert werden.

Zum Schutz des intrinsisch leitfähigen Polymers und zur Vermeidung von indifferenten Kriechströmen zwischen Elektrodenleitung 12 und dem angrenzenden Gewebe wird die Elektrodenleitung vorzugsweise durch eine Isolierhülle ummantelt.

### Bezugszeichenliste

- 10: Elektrostimulationsvorrichtung
- 12: Elektrodenleitung
- 14: Funktionselement
- 16: proximales Ende der Elektrodenleitung
- 18: distales Ende der Elektrodenleitung
- 20: erster Sektor
- 22: zweiter Sektor
- 24: erstes Kontaktelement
- 26: zweites Kontaktelement
- 28: erste Elektrode
- 30: zweite Elektrode
- 34: gemeinsames Kontaktelement für zwei Sektoren
- 36, 38: weitere Sektoren
- 40: Isolierhülle

## Patentansprüche

1. Elektrodenleitung für eine implantierbare, intravaskuläre Elektrostimulationsvorrichtung, wobei die Elektrodenleitung mit ihrem proximalen Ende mit der Elektrostimulationsvorrichtung und mit ihrem distalen Ende mit einem oder mehreren Funktionselementen elektrisch leitend verbunden ist, **dadurch gekennzeichnet, dass** die Elektrodenleitung (12) aus einem intrinsisch leitfähigen Polymer besteht, bei dem die einzelnen Polymerketten des Polymers so ausgerichtet sind, dass eine hohe elektrische Leitfähigkeit in axialer nicht jedoch in radialer Richtung der Elektrodenleitung (12) besteht (elektrische Anisotropie).

2. Elektrodenleitung nach Anspruch 1, **dadurch gekennzeichnet, dass** das Polymer aus Polyacetylenen (PAC), Polyparaphenylen (PPP), Polyphenylensulfid (PPS), Polyparaphenylvinylen (PPV), Polyphenylenbutadien (PPB), Polyparapyridin (PPYR), Polypyrrol (PPY), Polyfuran (PFU), Polythiophen (PT), Polyphenylamin (PANI), Polyethylendioxythiophen (PEDT), Polyethylendioxythophen-Polystyrolsulfonat (PEDT/PSS), Polyacen oder einer Kombination derselben besteht.

3. Elektrodenleitung nach Anspruch 1 oder 2, **dadurch gekennzeichnet, dass** ein spezifischer Durchgangswiderstand der Elektrodenleitung (12) in axialer Richtung 10⁻² bis 10⁻⁶ Ωm, insbesondere 10⁻⁴ bis 10⁻⁶ Ωm, beträgt.

4. Elektrodenleitung nach einem der Ansprüche 1 bis 3, **dadurch gekennzeichnet, dass** ein spezifischer Durchgangswiderstand der Elektrodenleitung (12) in radialer Richtung größer 5*10⁻¹ Ωm, insbesondere größer 1 Ωm, ist.

5. Elektrodenleitung nach einem der Ansprüche 1 bis 4, **dadurch gekennzeichnet, dass** die Funktionselemente (14) Elektroden (28, 30) zum Stimulieren oder Abfühlen von Körpergewebe umfassen.

6. Elektrodenleitung nach einem der Ansprüche 1 bis 4, **dadurch gekennzeichnet, dass** die Funktionselemente (14) Sensoren zur Erfassung physiologischer Parameter umfassen.

7. Elektrodenleitung nach einem der Ansprüche 1 bis 6, **dadurch gekennzeichnet, dass** die Elektrodenleitung (12) in ihrem Querschnitt in einzelne Sektoren (20, 22, 36, 38) gegliedert ist, die voneinander unabhängig in axialer Richtung elektrisch leitfähig sind.

8. Elektrodenfeitung nach Anspruch 7, **dadurch gekennzeichnet, dass** die Sektoren (20, 22, 36, 38) als Umlaufsegmente oder radiale Kreisausschnitte ausbildet sind.

9. Elektrodenleitung nach einem der Ansprüche 1 bis 8, **dadurch gekennzeichnet, dass** eine biokompatible Isolierhülle (40) die Elektrodenleitung (12) ummantelt.

10. Elektrodenleitung nach einem der Ansprüche 1 bis 8, **dadurch gekennzeichnet, dass** am proximalen Ende (16) der Elektrodenleitung (12) Kontaktelemente (24, 26, 34) der Elektrostimulationsvorrichtung (10) einem oder mehreren Sektoren (20, 22, 36, 38) zugeordnet sind.

11. Elektrodenleitung nach Anspruch 10, **dadurch gekennzeichnet, dass** ein einem oder mehreren Sektoren (20, 22, 36, 38) zugeordnetes Kontaktelement (24, 26, 34) den elektrischen Kontakt zu einem oder mehreren Funktionselementen (14) desgleichen beziehungsweise dergleichen Sektoren (20, 22, 36, 38) am distalen Ende (18) der Elektrodenleitung (12) gewährt.

## Claims

1. Electrode line for an implantable intravascular electrostimulation device, wherein the electrode line is electrically conductively connected with its proximal end to the electrostimulation device and with its distal end to one or more functional elements, **characterised in that** the electrode line (12) comprises an intrinsically conductive polymer in which the individual polymer chains of the polymer are so oriented that there is a high electrical conductivity in the axial direction of the electrode line (12) but not in the radial direction (electrical anisotropy).

2. Electrode line according to claim 1, **characterised in that** the polymer comprises polyacetylenes (PAC), polyparaphenylene (PPP), polyphenylene sulphide (PPS), polyparaphenylvinylene (PPV), polyphenylenebutadiene (PPB), polyparapyridine (PPYR), polypyrrole (PPY), polyfuran (PFU), polythiophene (PT), polyphenylamine (PANI), polyethylenedioxythiophene (PEDT), polyethylenedioxythiophene-polystyrene sulphonate (PEDT/PSS) and polyacene or a combination thereof.

3. Electrode line according to either claim 1 or claim 2, **characterised in that** a specific volume resistance of the electrode line (12) in the axial direction is between 10⁻² and 10⁻⁶ Ωm, in particular between 10⁻⁴ and 10⁻⁶ Ωm.

4. Electrode line according to any one of claims 1 to 3, **characterised in that** a specific volume resistance of the electrode line (12) in the radial direction is greater than 5* 10⁻¹ Ωm, in particular greater than 1 Ωm.

5. Electrode line according to any one of claims 1 to 4, **characterised in that** the functional elements (14) include electrodes (28, 30) for stimulating or sensing body tissue.

6. Electrode line according to any one of claims 1 to 4, **characterised in that** the functional elements (14) include sensors for detecting physiological parameters.

7. Electrode line according to any one of claims 1 to 6, **characterised in that** the electrode line (12) is divided in respect of its cross-section into individual sectors (20, 22, 36, 38) which are electrically conductive independently of each other in the axial direction.

8. Electrode line according to claim 7, **characterised in that** the sectors (20, 22, 36, 38) are in the form of peripheral segments or radial sectors of a circle.

9. Electrode line according to any one of claims 1 to 8, **characterised in that** a biocompatible insulating sheath (40) encases the electrode line (12).

10. Electrode line according to any one of claims 1 to 8, **characterised in that** at the proximal end (16) of the electrode line (12) contact elements (24, 26, 34) of the electrostimulation device (10) are associated with one or more sectors (20, 22, 36, 38).

11. Electrode line according to claim 10, **characterised in that** a contact element (24, 26, 34) associated with one or more sectors (20, 22, 36, 38) ensures electrical contact with respect to one or more functional elements (14) of the same sector or sectors (20, 22, 36, 38) at the distal end (18) of the electrode line (12).

## Revendications

1. Fil d'électrode pour un dispositif d'électrostimulation intravasculaire implantable, dans lequel le fil d'électrode est relié de façon électriquement conductrice par son extrémité proximale au dispositif d'électrostimulation et par son extrémité distale à un ou plusieurs éléments fonctionnels, **caractérisé en ce que** le fil d'électrode (12) est composé d'un polymère intrinsèquement conducteur, dans lequel les chaînes polymères individuelles du polymère sont orientées de sorte qu'une conductivité électrique élevée existe dans la direction axiale mais pas dans la direction radiale du fil d'électrode (12) (anisotropie électrique).

2. Fil d'électrode selon la revendication 1, **caractérisé en ce que** le polymère est constituée de polyacétylène (PAC), polyparaphénylène (PPP), polysulfure de phénylène (PPS), polyparaphénylvinylène (PPV), polyphénylènebutadiène (PPB), polyparapyridine (PPYR), polypyrrole (PPY), polyfurane (PFU), polythiophène (PT), polyphénylamine (PANI), polyéthylènedioxythiophène (PEUT), polyéthylènedioxythiophène-polystyrol sulfonate (PEDT/PSS), polyacène ou d'une combinaison de ceux-ci.

3. Fil d'électrode selon la revendication 1 ou 2, **caractérisé en ce que** la résistivité transversale du fil d'électrode (12) atteint dans la direction axiale 10⁻² à 10⁻⁶ Ωm, en particulier 10⁻⁴ à 10⁻⁶ Ωm.

4. Fil d'électrode selon l'une des revendications 1 à 3, **caractérisé en ce qu'**une résistivité transversale du fil d'électrode (12) est supérieure à 5*10⁻¹ Ωm, en particulier supérieure à 1 Ωm dans la direction radiale.

5. Fil d'électrode selon l'une des revendications 1 à 4, **caractérisé en ce que** les éléments fonctionnels (14) comprennent les électrodes (28, 30) en vue de la stimulation ou de la détection de tissus corporels.

6. Fil d'électrode selon l'une des revendications 1 à 4, **caractérisé en ce que** les éléments fonctionnels (14) comprennent des capteurs en vue de la détection de paramètres physiologiques.

7. Fil d'électrode selon l'une des revendications 1 à 6, **caractérisé en ce que** le fil d'électrode (12) est séparé en coupe en secteurs individuels (20, 22, 36, 38), qui sont électriquement conducteurs dans la direction axiale indépendamment l'un de l'autre.

8. Fil d'électrode selon la revendication 7, **caractérisé en ce que** les secteurs (20, 22, 36, 38) sont réalisés en tant que segments de circulation ou secteurs circulaires radiaux.

9. Fil d'électrode selon l'une des revendications 1 à 8, **caractérisé en ce qu'**une enveloppe isolante biocompatible (40) entoure le fil d'électrode (12).

10. Fil d'électrode selon l'une des revendications 1 à 8, **caractérisé en ce que**, à l'extrémité proximale (16) du fil d'électrode (12), des éléments de contact (24, 26, 34) du dispositif d'électrostimulation (10) sont associés à un ou plusieurs secteurs (20, 22, 36, 38).

11. Fil d'électrode selon la revendication 10, **caractérisé en ce qu'**un élément de contact (24, 26, 34) associé à un ou plusieurs secteurs (20, 22, 36, 38) garantit le contact électrique à un ou plusieurs éléments fonctionnels (14) du même ou des mêmes secteurs (20, 22, 36, 38) à l'extrémité distale (18) du fil d'électrode (12).
